# EUROPÄISCHE PATENTSCHRIFT

(11) **EP 2 438 230 B1**
(45) Veröffentlichungstag und Bekanntmachung des Hinweises auf die Patenterteilung: **09.10.2013**
(21) Anmeldenummer: 10722687.0
(22) Anmeldetag: 28.05.2010
(51) Int. Cl.: D06F 39/00, A47L 15/42

(54) **HAUSGERÄT MIT EINER OBERFLÄCHE, WELCHE EINEN PHOTOKATALYSATOR AUFWEIST**
DOMESTIC APPLIANCE HAVING A SURFACE WHICH COMPRISES A PHOTOCATALYST
APPAREIL MÉNAGER POURVU D'UNE SURFACE COMPRENANT UN PHOTOCATALYSEUR

(30) Priorität: 04.06.2009 DE 102009026712
(43) Veröffentlichungstag der Anmeldung: 11.04.2012
(73) Patentinhaber: BSH Bosch und Siemens Hausgeräte GmbH, 81739 München (DE)
(72) Erfinder: HANAU, Andreas, 12359 Berlin (DE); SCHULZE, Ingo, 16341 Panketal (DE); EGLMEIER, Hans, 10587 Berlin (DE); SCHAUB, Hartmut, 14656 Brieselang (DE)
(86) Internationale Anmeldenummer: PCT/EP2010/057411
(87) Internationale Veröffentlichungsnummer: WO 2010/139623

(56) Entgegenhaltungen:
- EP-A1- 1 602 774
- WO-A2-2008/052975
- JP-A- 11 123 299

## Beschreibung

Die Erfindung betrifft ein Hausgerät mit mindestens einem Bauteil, welches eine durch eine organische Verschmutzung belastbare Oberfläche aufweist, welche Oberfläche einen Photokatalysator aufweist und welcher Oberfläche eine Photoquelle zum Bestrahlen des Photokatalysators mit einer aktivierenden elektromagnetischen Strahlung zugeordnet ist.

Ein solches Hausgerät geht hervor aus jeder der Veröffentlichungen JP 11-123299 A, JP 11-137894 A und JP 11-137881 A. Demgemäß sind in einer Waschmaschine ein Laugenbehälter und ein darin um eine senkrechte Achse drehbarer Wäschekorb mit Schichten eines Photokatalysators beschichtet, und es ist eine bewegliche Ultraviolett-Photoquelle vorgesehen, mittels welcher die Schichten zur Aktivierung des Photokatalysators mit ultraviolettem Licht bestrahlbar sind. Teile des Waschkorbs, die eine Schicht des Photokatalysators von der Photoquelle abschatten könnten, können aus für die Strahlung transparentem Material bestehen.

In einem wasserführenden Hausgerät werden im Allgemeinen Gegenstände, die auf unterschiedliche Weise verunreinigt sind, gereinigt. So fallen als Verunreinigungen in einer Geschirrspülmaschine Essensreste an, und das Spektrum an Verunreinigungen von in einer Waschmaschine zu reinigenden Wäschestücken ist in der Regel größer. Allen wasserführenden Hausgeräten ist gemeinsam, dass darin, insbesondere an weniger gut zugänglichen Stellen, Verunreinigungen ausfallen und sich anreichern können. Solche Verunreinigungen können in einer in einem entsprechenden Hausgerät vorherrschenden feuchtwarmen Atmosphäre ein guter Nährboden für Mikroorganismen wie Bakterien und Pilze sein und damit zusätzliche Verunreinigungen hervorrufen.

So kann es in einem Wäschebehandlungsgerät wie beispielsweise einer Waschmaschine bei einer häufigen Anwendung von Waschprogrammen mit kalten Laugen, in einer langen Stillstandphase mit geschlossener Tür oder unter ungünstigen Aufstellbedingungen aufgrund von Mikroorganismen zu Geruchsbeeinträchtigungen und/oder sichtbaren Verunreinigungen kommen. Wünschenswert ist daher eine Möglichkeit zu einer Reinigung auch solcher schlecht zugänglichen Bereiche. Überdies ist es wünschenswert, dass vorbeugende Maßnahmen gegen Mikroorganismen selbst bei äußerlich nicht wahrnehmbaren Effekten ergriffen werden können. Insgesamt ist es sehr sinnvoll, dass solche störenden Verunreinigungen vermieden bzw. weitestgehend eliminiert werden können.

Aus dem Stand der Technik sind verschiedene Maßnahmen gegen solche Verunreinigungen bekannt. Insbesondere werden bisweilen Gerätereinigungsprogramme angeboten, die bei hohen Temperaturen mit Unterstützung von Waschmitteln und teilweise unter erhöhten Flottenständen und/oder bei höheren Trommeldrehzahlen, d.h. bei einem erhöhten Eintrag mechanischer Energie, entstandene Verschmutzungen wieder beseitigen. Bekannt sind außerdem die Verwendung von Ozon sowie UV-C-Strahlern zur Entfernung organischer Verschmutzungen. Bekannt sind ebenfalls Maßnahmen zur Abtötung von Mikroorganismen unter Verwendung von Ag⁺- oder Cu⁺-Ionen in einer Waschflotte bzw. auf den Oberflächen der mit Waschflotte in Berührung stehenden Materialien. Andere Verfahren zielen auf die thermische Abtötung eventuell vorhandener Mikroorganismen durch die Erhöhung der Temperaturen an den Oberflächen der mit der Waschflotte in Berührung stehenden Bauteile über eine direkte oder indirekte Energieübertragung (Wasser, Dampf, Mikrowelle). Bekannt aus den eingangs zitierten Dokumenten ist schließlich auch der Einsatz von Photokatalysatoren. Nachteilig bei diesen bekannten Verfahren und Maßnahmen sind teilweise hohe Apparate- und/oder Betriebskosten, um tatsächlich relevante Effekte zu erzielen. Im Falle des Einsatzes von Ag⁺- und Cu⁺-Ionen kommen nachteilige Auswirkungen hinsichtlich Boden- und Gewässerbelastungen hinzu.

Aus den Schriften WO 2005/108505 A1, EP 1 205 244 A1 und EP 1 205 245 A1 sind Mineralien bekannt, deren Oberflächen unter Bestrahlung mit sichtbarem oder ultraviolettem Licht Moleküle des Sauerstoffs derart zu aktivieren vermögen, dass ihre Bereitschaft zu chemischen Reaktionen deutlich erhöht wird. Dieser Effekt ist unter der Bezeichnung "Photokatalyse" geläufig. Jedes dieser Mineralien ist ein Halbleiter mit einer Bandlücke, welche die Erzeugung eines Elektron-Loch-Paars in diesem Halbleiter durch ein Quant sichtbaren oder ultravioletten Lichts erlaubt. Zusätzlich ist davon auszugehen, dass ein solches Mineral eine Affinität zu Sauerstoff aufweist, welche eine Aktivierung des Sauerstoffs durch Übertragung von Energie aus dem Halbleiter gestattet. Zu den bekannten Mineralien gehören Titandioxid, Cadmiumsulfid, Wolframtrioxid und Zinkoxid. Zusätzlich ist es bekannt, einen solchen Halbleiter durch Dotieren oder dergleichen Modifizieren mit Elementen wie Kohlenstoff, Bor, Stickstoff, Phosphor, Schwefel, Chlor, Arsen, Selen, Brom, Antimon, Tellur und Jod dahingehend zu verändern, dass die Wellenlänge des zum Hervorrufen der Photokatalyse notwendigen Lichtes verlängert wird. Als Beispiel sei Titandioxid erwähnt: Zum Hervorrufen der Photokatalyse an reinem Titandioxid ist eine Bestrahlung mit ultraviolettem Licht einer Wellenlänge zwischen 380 nm und 400 nm entsprechend dem Bereich UV-A erforderlich. Gemäß der Veröffentlichung WO 2005/108505 A1 ist es möglich, ein mit Kohlenstoff modifiziertes Titandioxid in Form eines Pulvers zu erzeugen, an dem bereits bei Bestrahlen mit sichtbarem Licht die Photokatalyse auftritt. Dieser pulverförmige Photokatalysator ist verwendbar in Bauteilen aus Beton, Keramik, Papier und Geweben in der Bauindustrie und Fahrzeugindustrie, sowie in Photovoltaikzellen, Klimaanlagen und Apparaten zur Reinigung oder Sterilisierung von Luft oder Wasser.

Von den bekannten Materialien, die unter entsprechenden Bedingungen Photokatalyse zeigen, ist Titandioxid besonders ausgezeichnet dadurch, dass es chemisch weitgehend inert und ungiftig sowie in vielfältigen Verwendungen, insbesondere als Weißpigment, bekannt ist und in großem industriellem Maßstabe produziert und vermarktet wird.

Aufgabe der vorliegenden Erfindung ist es daher, ein Hausgerät der eingangs definierten Gattung bereitzustellen, in dem bzw. mit dem auf einfache und besonders hygienische Weise Verunreinigungen beseitigt oder verhindert oder zumindest weitgehend beseitigt oder verhindert werden können. Insbesondere sollen Verunreinigungen mit Mikroorganismen beseitigt oder verhindert werden können.

Die Lösung dieser Aufgabe wird nach dieser Erfindung erreicht durch das Hausgerät mit den Merkmalen des entsprechenden unabhängigen Patentanspruchs. Bevorzugte Ausführungsformen des erfindungsgemäßen Hausgeräts sind in entsprechenden abhängigen Patentansprüchen aufgeführt. Bevorzugten Ausführungen des Hausgeräts entsprechen bevorzugte Ausführungen des Verfahrens und umgekehrt, und dies auch dann, wenn darauf hierin nicht explizit hingewiesen ist.

Gegenstand der Erfindung ist somit ein Hausgerät mit mindestens einem Bauteil, welches eine durch eine organische Verschmutzung belastbare Oberfläche aufweist, welche Oberfläche einen Photokatalysator aufweist und welcher Oberfläche eine Photoquelle zum Bestrahlen des Photokatalysators mit einer aktivierenden elektromagnetischen Strahlung zugeordnet ist; wobei die Oberfläche von einem urgeformten ersten Werkstoff gebildet ist, in welchem der Photokatalysator dispergiert ist.

Im erfindungsgemäßen Hausgerät können grundsätzlich beliebige innere Oberflächen einen Photokatalysator aufweisen.

In optisch schwer zugänglichen Bereichen eines erfindungsgemäßen Hausgeräts (z.B. der Bereich des Laugenbehälters hinter der Trommelrückwand in einer entsprechenden Waschmaschine) kann mit zusätzlich angeordneten Photoquellen oder entsprechend ausgebildeten Vorrichtungen für die optische Umlenkung der elektromagnetischen Strahlung unter Ausnutzung von Spiegelflächen (z.B. der Innentrommelrückwand) eine flächige Ausleuchtung erreicht werden. Bei einer entsprechenden Ausstattung der inneren Flächen des Hausgerätes mit dem Photokatalysator kann so eine besonders effiziente Reinigung erreicht werden.

In einer bevorzugten Ausführung des erfindungsgemäßen Hausgeräts ist der Photokatalysator ein Füllstoff in dem ersten Werkstoff; mit weiterem Vorzug ist der erste Werkstoff ein mit dem Füllstoff gefüllter erster polymerer Werkstoff, mit noch weiterem Vorzug ein mit dem Füllstoff gefüllter Thermoplast. Insbesondere kann der erste Werkstoff durch Spritzgießen verarbeitet werden. Dabei bildet der Thermoplast eine kontinuierliche Matrix, in der der Füllstoff dispergiert ist. Insoweit der Füllstoff an der Oberfläche vorliegt, ist er der Bestrahlung durch die Photoquelle bei gleichzeitiger Exposition an Sauerstoff zugänglich und kann seine katalytische Wirkung entfalten, wodurch er beiträgt zur Erzeugung von aktiviertem Sauerstoff, der zum Beseitigen unerwünschter Ablagerungen sowie zum Desinfizieren durch Oxidation geeignet ist.

In einer weiteren bevorzugten Ausführung des erfindungsgemäßen Hausgeräts bildet der erste Werkstoff eine Schicht an der Oberfläche und überdeckt einen zweiten Werkstoff. Der erfindungsgemäß als Photokatalysator nutzbare Füllstoff im ersten Werkstoff ist fern von der Oberfläche ohnehin nicht katalytisch aktiv; es ist dementsprechend denkbar, abseits der Oberfläche einen zweiten Werkstoff zu verwenden, welcher insbesondere einen anderen Füllstoff aufweisen und im übrigen dem ersten Werkstoff gleich oder ähnlich sein kann. Ein entsprechendes Bauteil kann beispielsweise durch ein zweistufiges Spritzgießen erzeugt werden, wobei zunächst der erste Werkstoff und dann der zweite Werkstoff gespritzt wird, so dass der erste Werkstoff die Oberfläche bildet und der zweite Werkstoff die zum Spritzgießen verwendete Form erst vollständig füllt. Im zweiten Werkstoff kann insbesondere ein gegenüber dem Photokatalysator billigerer Füllstoff, beispielsweise Talkum, vorhanden sein. Der zweite Werkstoff kann gleiche oder andere mechanische Eigenschaften wie bzw. als der erste Werkstoff aufweisen.

In einer besonders bevorzugten Ausführung des erfindungsgemäßen Hausgeräts ist der Photokatalysator ein halbleitendes Mineral, insbesondere Titandioxid, Cadmiumsulfid, Wolframtrioxid oder Zinkoxid. Dieses Mineral kann insbesondere zur Ermöglichung der Photokatalyse bei Bestrahlung mit sichtbarem Licht modifiziert sein durch einen Zusatz von Kohlenstoff, Bor, Stickstoff, Phosphor, Schwefel, Chlor, Arsen, Selen, Brom, Antimon, Tellur oder Jod.

Ganz besonders bevorzugt im Rahmen der Erfindung ist die Nutzung von Titandioxid als Füllstoff. Titandioxid kommt in verschiedenen Formen vor. Für die vorliegende Erfindung hat es sich als besonders vorteilhaft erwiesen, wenn Titandioxid in der Modifikation von Anatas vorliegt. Damit ist aber kein Ausschluss anderer Modifikationen des Titandioxids, namentlich Rutil und Brookit, zur Verwendung im Rahmen der Erfindung verbunden. Ebenfalls ganz besonders bevorzugt ist ein Titandioxid, welches mittels einer Dotierung aus Kohlenstoff modifiziert ist. Es sei angemerkt, dass durch eine solche Modifizierung insbesondere ein eventueller Nachteil der Rutil- oder Brookit-Modifikation des Titandioxids ausgeglichen werden könnte.

Das den Photokatalysator bildende Mineral liegt in der Oberfläche vorzugsweise in Form von Nanopartikeln vor. Besonders vorteilhaft besteht das Mineral aus Partikeln mit einem mittleren Durchmesser d kleiner oder gleich 10 nm, insbesondere aus Partikeln mit einem mittleren Durchmesser d kleiner oder gleich 8 nm.

In einer bevorzugten Ausführung weist das erfindungsgemäße Hausgerät eine Photoquelle auf, die eingerichtet ist zum Aussenden der elektromagnetischen Strahlung in einem Wellenlängenbereich umfassend sichtbares Licht und ultraviolettes Licht. Insbesondere ist die Photoquelle eingerichtet zum Aussenden der elektromagnetischen Strahlung im Wellenlängenbereich von 380 nm bis 400 nm.

In einer bevorzugten Ausführung des erfindungsgemäßen Hausgeräts ist die Photoquelle eine Anordnung mit zumindest einer Leuchtdiode. In einer besonders bevorzugten Ausführungsform weist das Hausgerät eine solche Anordnung mit mindestens sechs Leuchtdioden auf, die elektromagnetische Strahlung im Bereich von 380 bis 400 nm emittieren.

Es können erfindungsgemäß beliebige Leuchtdioden eingesetzt werden, wobei diese insbesondere elektromagnetische Strahlung im Bereich von 380 bis 400 nm und insbesondere im Bereich von 385 bis 395 nm emittieren, um den gewünschten Effekt am verwendeten Photokatalysator hervorzurufen. Es ist hierzu nicht erforderlich, dass die Wellenlänge eines Maximums in einer Intensitätsverteilung einer solchen Leuchtdiode in diesem Wellenlängenbereich liegt. Es genügen bereits Anteile an elektromagnetischer Strahlung im jeweils gewünschten Bereich der Wellenlänge, wobei vorzugsweise Dioden mit einem großen Strahlungsanteil in diesem Bereich eingesetzt werden. Zum Aussenden von UV-Strahlung geeignete Leuchtdioden sind aufgebaut auf Halbleitern wie Galliumnitrid, Indium-Gallium-Nitrid, Zinkselenid und Siliciumcarbid. Derartige Leuchtdioden sowie Arrays aus solchen, das sind fertig und als bauliche Einheiten konfektionierte Anordnungen solcher Leuchtdioden, sind kommerziell erhältlich.

Das erfindungsgemäße Hausgerät ist hinsichtlich seiner bestimmungsgemäßen Verwendung grundsätzlich nicht eingeschränkt. So kommen z.B. Kühlschränke, Herde, Mikrowellengeräte etc. in Frage. Vorzugsweise ist das Hausgerät ein wasserführendes Hausgerät, insbesondere ein Geschirrspülgerät oder ein Wäschebehandlungsgerät. Das Hausgerät ist besonders bevorzugt ein Wäschebehandlungsgerät aus der Gruppe Waschmaschine, Trockner und Waschtrockner. Im Allgemeinen weist ein Wäschebehandlungsgerät eine drehbar gelagerte Trommel, einen Antriebsmotor für die Trommel und eine Heizeinrichtung auf. Zudem sind in der Regel Schaltmittel und Antriebsmittel zum Drehen und Anhalten der Trommel vorhanden.

Ein Trockner umfasst im Allgemeinen einen Prozessluftkanal, in dem sich eine Trocknungskammer für die zu trocknenden Gegenstände und im Allgemeinen eine Heizung zur Erwärmung der Prozessluft und ein Gebläse zur Beförderung der Prozessluft befinden. Ein Trockner wird im Allgemeinen als Umluft- oder als Ablufttrockner betrieben, wobei auch Mischformen bekannt sind. In einem Umlufttrockner wird zum Trocknen von Gut wie Wäsche Prozessluft in einem geschlossenen Kreislauf durch eine Trocknungskammer mit dem zu trocknenden Gut geführt. Aus der Trocknungskammer strömende feuchtwarme Prozessluft wird zum Auskondensieren der von dem feuchten Gut herrührenden Feuchte in einem geeigneten Wärmetauscher (Luft-Luft-Wärmetauscher, Wärmesenke einer Wärmepumpe) abgekühlt und nach Abtrennen des entstandenen Kondensats wieder mittels einer Heizung (elektrische Heizung, Gasheizung oder Wärmequelle einer Wärmepumpe) erhitzt und in die Trocknungskammer zurück geleitet. In einem Ablufttrockner sind dagegen ein Zuluftkanal und ein Abluftkanal vorhanden, so dass die aus einer Umgebung des Ablufttrockners (insbesondere einem Aufstellraum) in den Zuluftkanal gesaugte Prozessluft nach Durchgang durch die Trocknungskammer über den Abluftkanal zu einem Abluftausgang und damit wieder in den Aufstellraum, oder durch eine entsprechende Abluftleitung aus diesem heraus, geführt wird.

Eine Waschmaschine umfasst im Allgemeinen neben einer Trommel als Aufnahmebehälter für die zu behandelnden Wäschestücke einen Laugenbehälter, ein Wasserzulaufsystem und ein am Boden des Laugenbehälters angeordnetes Laugenablaufsystem mit einer Laugenpumpe. Ein Waschtrockner vereinigt die Merkmale eines Trockners und einer Waschmaschine in sich und ist für beide entsprechenden Verwendungen nutzbar.

Ist das erfindungsgemäße Hausgerät ein Wäschebehandlungsgerät, so weist dieses insbesondere Wäschemitnehmer, eine Manschette, einen Laugenbehälter und/oder ein sonstiges der zu behandelnden Wäsche ausgesetztes und insbesondere als Ganzes urgeformtes Bauteil mit Oberfläche, die den Photokatalysator enthält, auf.

In einer bevorzugten Ausführung eines erfindungsgemäßen wasserführenden Hausgeräts, welches zumindest ein mit einer Waschlauge beaufschlagbares urgeformtes Bauteil aufweist, hat dieses Bauteil die Oberfläche, in der der Photokatalysator dispergiert ist. Auf diese Weise ist das Bauteil einer Reinigung durch aktivierten Sauerstoff durch Vermittlung des erfindungsgemäß vorgesehenen Photokatalysators zugänglich. Das Bauteil ist weiter vorzugsweise ausgewählt aus der Gruppe umfassend Manschette, Wäschemitnehmer und Laugenbehälter.

Im Falle des Vorliegens des Photokatalysators auf einem Mitnehmer oder einem anderen direkt der Wäsche ausgesetzten Bauteil einer Waschmaschine, eines Trockners oder eines Waschtrockners können Geruch beseitigende oder bleichende Effekte an der in der Innentrommel bewegten Wäsche nutzbar sein. Dies kann dadurch geschehen, dass bei Kontakt mit den Titandioxid beschichteten Oberflächen bei unmittelbar zuvor oder zeitgleich in unmittelbarer Nachbarschaft zur Kontaktfläche aktivierter Photokatalyse erzeugter aktivierter Sauerstoff gleichermaßen auf den angrenzenden nicht behandelten Oberflächen wirkt.

In einer ebenfalls bevorzugten Ausführung des erfindungsgemäßen Hausgeräts ist die Photoquelle im Bereich einer Zugangsöffnung angeordnet, wobei die Zugangsöffnung beispielsweise ein Bullauge einer entsprechenden Waschmaschine sein kann. Die Photoquelle kann dabei vorteilhafterweise eine Beleuchtung sein, die einen Arbeitsbereich des Hausgeräts, beispielsweise das Innere der Wäschetrommel einer Waschmaschine, beleuchtet und einem Benutzer einen Einblick in das Innere des arbeitenden Hausgeräts gestattet. Dabei verbindet die Photoquelle die Nutzung zur Aktivierung des Photokatalysators mit der Nutzung zur Anzeige des Inbetriebseins des Hausgeräts und der Nutzung zur Beleuchtung seines Arbeitsbereiches für einen Nutzer, der einen Einblick zu nehmen wünscht. Die Erfindung eignet sich somit insbesondere für den Einsatz bei Hausgeräten, die bereits über elektrische Beleuchtungseinrichtungen in ihrem Inneren verfügen. Die erfindungsgemäß vorgesehene Photoquelle lässt sich vorzugsweise in Kombination, an Stelle oder alternierend zu einer vorhandenen Innenraumbeleuchtung betreiben. Sie kann aber auch an beliebigen sonstigen geeigneten Stellen im Innenraum und/oder schlecht belüfteten Bereichen eines Hausgerätes angeordnet sein.

In Ausführungsformen, bei denen im Hausgerät noch weitere Photoquellen vorhanden sind, die ggf. im sichtbaren Teil des Spektrums Strahlung emittieren, können diese ggf. zur Aktivierung des Photokatalysators zugeschaltet werden. Dabei können ggf. verschiedene Lichteffekte erzeugt werden. Beispielsweise könnte beim Start einer erfindungsgemäßen Reinigung ein spezielles Beleuchtungsprogramm mit sich ändernden Farben gestartet werden, das anzeigt, dass gerade die Reinigung durchgeführt wird. Alternativ oder in Ergänzung hierzu können bestimmte Geräusche oder Töne erzeugt werden, die anzeigen, dass gerade die Reinigung durchgeführt wird.

In einer bevorzugten Ausführungsform der Erfindung wird im Bereich einer Trommelinnenbeleuchtung einer Waschmaschine eine UV-A-Leuchtdiode oder ein UV-A-Leuchtdiodenarray eingesetzt. Die davon angestrahlten Bereiche im Laugenbehälter und der Manschette der Waschmaschine weisen eine Titandioxid enthaltende Schicht auf. Die Erzeugung der Titandioxid enthaltenden Schicht geschieht in der Regel beim Herstellungsprozess des Bauteils. Das im Bereich von Laugenbehältern aus Kunststoff üblicherweise mit Talkum oder Glasfaser verstärkte Polypropylen lässt sich zusätzlich oder alternativ im Spritzguss-Prozess mit Titandioxid anreichern, so dass auf einer inneren Fläche des Laugenbehälters eine Titandioxid enthaltende Schicht gebildet wird. In ähnlicher Weise kann diese bei einer EPDM-Manschette im Vulkanisationsprozess geschehen.

In einer besonders bevorzugten Ausführungsform der Erfindung weist das Hausgerät einen optischen Sensor mit einer Lichtquelle und einem Lichtempfänger auf, wobei die Lichtquelle die Photoquelle ist. Somit erfüllt auch ein dieser Ausführung die Photoquelle eine mehrfache Funktion. Dabei ist es natürlich notwendig, dass der Photokatalysator in einer Umgebung des Sensors vorliegt und von der von der Photoquelle ausgesendeten Strahlung erreichbar ist.

In einer ebenfalls besonders bevorzugten Ausführungsform weist das erfindungsgemäße Hausgerät einen Geruchssensor auf. Ein solcherart vorgesehener Geruchssensor kann an verschiedenen Stellen angeordnet sein. In einem als Trockner ausgestalteten erfindungsgemäßen Hausgerät ist der Geruchssensor vorzugsweise in der Trocknungskammer und/oder im Prozessluftkanal hinter der Trocknungskammer angeordnet. Ist der Trockner ein Kondensationstrockner, empfiehlt sich beispielsweise eine Anordnung des Geruchssensors hinter einem Wärmetauscher, in dem die Feuchtigkeit der feuchtwarmen Prozessluft kondensieren kann. Hierdurch kann der Einfluss der Feuchtigkeit auf das Sensorsignal des Geruchssensors minimiert werden. In einem als Waschmaschine ausgestalteten erfindungsgemäßen Hausgerät ist der Geruchssensor vorzugsweise in der Trommel angeordnet. Dies ermöglicht eine Registrierung von Geruchsstoffen vor Durchführung eines Waschverfahrens, so dass noch vor Waschbeginn eine Reinigung durchgeführt werden kann. Bei Verwendung in einer Waschmaschine kann der Geruchssensor vor Einleitung von Wasser bzw. Waschlauge ggf. über eine verschließbare Abdeckung vor schädlichen Einflüssen durch Wasser bzw. die Waschlauge gesichert werden.

Gegenstand der Erfindung ist außerdem ein Verfahren zur Reinigung einer durch eine organische Verschmutzung belastbare Oberfläche mindestens eines Bauteils eines Hausgeräts, in welcher Oberfläche ein Photokatalysator dispergiert und welcher Oberfläche eine Photoquelle zum Bestrahlen des Photokatalysators mit einer aktivierenden elektromagnetischen Strahlung zugeordnet ist, und wobei die Oberfläche von einem urgeformten ersten Werkstoff gebildet ist, bei welchem Verfahren der Photokatalysator von der Photoquelle durch Bestrahlen mit elektromagnetischer Strahlung aktiviert wird.

In Ausführungsformen des erfindungsgemäßen Verfahrens kann die Reinigung manuell oder automatisch gestartet werden. Der automatische Start kann z.B. bei einem Wäschebehandlungsprogramm vor Beginn, während oder nach Beendigung des Wäschebehandlungsprogramms durchgeführt werden.

Alternativ kann der Beginn eines erfindungsgemäßen Reinigungsverfahrens auch an die Erkennung von Verunreinigungen und/oder schlechten Gerüchen gekoppelt sein. So ist bei einer Ausführungsform der Erfindung im Hausgerät ein Geruchssensor vorhanden. Nach Feststellung eines schlechten Geruches durch den Geruchssensor kann dann das erfindungsgemäße Verfahren begonnen werden, wobei der Zeitpunkt für den Verfahrensbeginn unterschiedlich gewählt sein kann. Das Reinigungsverfahren kann sofort und prinzipiell vor, während und nach z.B. einem Wäschebehandlungsprogramm durchgeführt werden. Zur Erkennung von schlechten Gerüchen können in einer Programmsteuerung des Hausgeräts geeignete Referenzsignale hinterlegt sein. Erfindungsgemäß ist es bevorzugt, dass beim Nachweis eines vorgegebenen Geruchsstoffes bzw. einer vorgegebenen Geruchsstoffmischung oder beim Überschreiten eines bestimmten Schwellenwertes für die Konzentration eines vorgegebenen Geruchsstoffes oder einer vorgegebenen Geruchsstoffmischung ein erfindungsgemäßes Verfahren zur Reinigung eines Hausgeräts durchgeführt wird.

Die Dauer des erfindungsgemäßen Verfahrens kann fallweise unterschiedlich und an die Erfüllung verschiedener Voraussetzungen gebunden sein. So können Zeitpunkt und Dauer des erfindungsgemäßen Verfahrens von einem seit Durchführung eines letzten Reinigungsverfahrens oder Wäschebehandlungsprogramms verstrichenen Zeitraum abhängen. Hierzu kann in einer Programmsteuerung des Hausgeräts eine geeignete Uhr vorhanden sein.

In einer bevorzugten Ausführungsform des erfindungsgemäßen Verfahrens wird die Durchführung des erfindungsgemäßen Reinigungsverfahrens durch ein akustisches und / oder optisches Signal angezeigt. Erfindungsgemäß ist es daher bevorzugt, dass die Anwesenheit eines Geruchsstoffs mittels einer Anzeigevorrichtung des Hausgeräts angezeigt wird. Dies kann über eine akustische und/oder optische Anzeigevorrichtung geschehen.

Die Erfindung hat den Vorteil, dass auf einfache und kostengünstige Weise ein Hausgerät nebst zugehörigem Verfahren zu seiner Reinigung zur Verfügung gestellt wird, das deutlich weniger zu Verunreinigungen, insbesondere auch mit Mikroorganismen, neigt und bei dennoch auftretenden Verunreinigungen leicht zu reinigen ist.

Das erfindungsgemäße Hausgerät und das in ihm ausübbare erfindungsgemäße Verfahren haben den Vorteil, dass auf einfache und automatische Weise eine Reinigung durchgeführt werden kann. Dies kann automatisch z.B. vor Durchführung oder nach Beendigung eines Wäschebehandlungsprogramms oder aber nach Feststellung von Verunreinigungen (z.B. Geruchsstoffen) durchgeführt werden. Auf diese Weise können Wäschebehandlungsprogramme bzw. Reinigungsprogramme auf Art und Menge von ggf. vorhandenen organischen Verbindungen und insbesondere von Mikroorganismen abgestimmt werden.

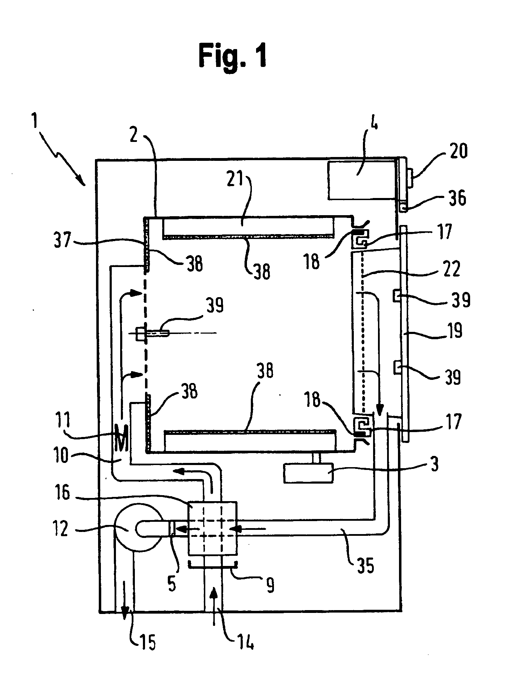

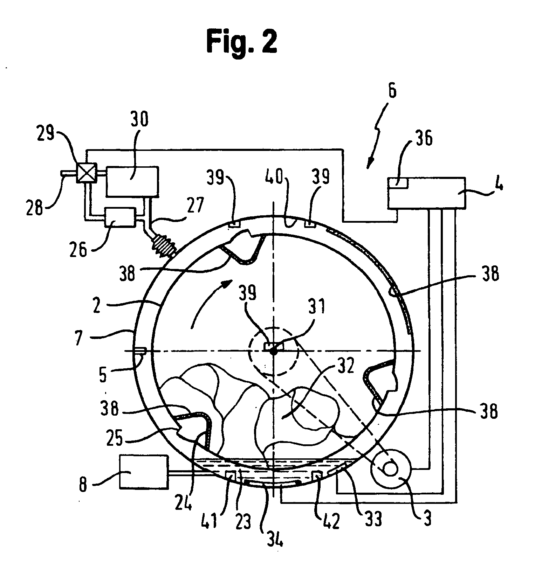

Weitere Einzelheiten der Erfindung ergeben sich aus der nachfolgenden Beschreibung von nicht einschränkenden Ausführungsbeispielen für das erfindungsgemäße Hausgerät und das erfindungsgemäße Verfahren zur Reinigung eines Hausgeräts. Hierbei wird Bezug genommen auf die Figuren 1 und 2, in denen als Hausgerät jeweils ein Wäschebehandlungsgerät dargestellt ist. Andere Ausführungsformen sind denkbar. Im Einzelnen zeigen:
Fig. 1 einen vertikalen Schnitt durch eine als Ablufttrockner ausgestaltete erste Ausführungsform eines Hausgeräts;
Fig. 2 schematisch einen vertikalen Schnitt durch eine als Waschmaschine ausgestaltete zweite Ausführungsform des Hausgeräts, und
Figur 3 einen Schnitt durch ein urgeformtes Bauteil.

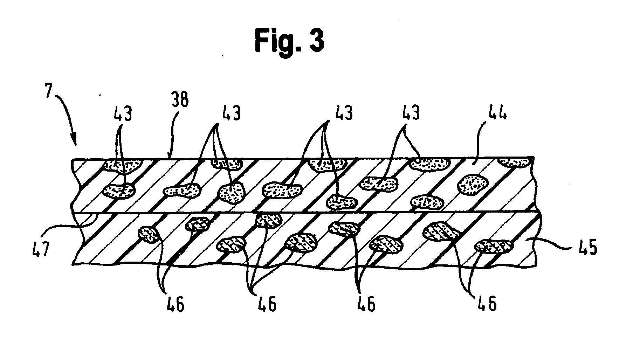

Der in Fig. 1 dargestellte Ablufttrockner 1 weist eine um eine horizontale Achse drehbare Trommel 2 als Trocknungskammer auf, innerhalb welcher Mitnehmer 21 zur Bewegung von Wäsche während einer Trommeldrehung befestigt sind. Diese Mitnehmer 21 sind urgeformt, konkret spritzgegossen, aus einem thermoplastischen Polymer, welches Titandioxid in der Anatas-Modifikation als Füllstoff aufweist (zu dem Photokatalysator und dem Polymer vgl. Bezugszeichen 43 und 44 in Figur 3, welche weiter unten erläutert ist). Das Titandioxid ist in Pulverform hergestellt und mit dem Polymer verbunden worden. Es kann mit Kohlenstoff dotiert sein, um durch sichtbares Licht als Photokatalysator aktivierbar zu sein. Es wird bemerkt, dass die Verwendung von Titandioxid als Füllstoff durchaus geläufig ist, wobei dieser Füllstoff allerdings gelegentlich chemisch deaktiviert wird, um die photokatalytische Wirkung, die je nach Anwendung anders als hier durchaus unerwünscht sein kann, zu vermeiden. Vorliegend muss also darauf geachtet werden, dass nicht etwa ein solcherart deaktiviertes Titandioxid zur Verwendung kommt.

Zum Betreiben des Ablufttrockners 1 wird Prozessluft mit Hilfe des Gebläses 12 von einem Zulufteingang 14 in einem Zuluftkanal 10 über eine Heizung 11 durch die Trommel 2 mit der zu trocknenden Wäsche und einen Abluftkanal 35 zu einem Abluftausgang 15 geführt. Bei diesem Ablufttrockner bilden somit Zuluftkanal 10, Trommel 2 und Abluftkanal 35 einen Prozessluftkanal. Von der Heizung 11 erwärmte Luft wird durch die gelochte Fläche der Trommelrückwand 37 in die Trommel 2 geleitet, kommt dort mit der zu trocknenden Wäsche in Berührung und strömt durch die Befüllöffnung der Trommel 2 zu einem Flusensieb 22 innerhalb der die Befüllöffnung verschließenden Tür 19. Anschließend wird die feucht-warme Prozessluft in der Tür 19 nach unten umgelenkt. Die Prozessluft wird im Abluftkanal 35 einem Luft-Luft-Wärmetauscher 16 zugeführt, in dem die warme, mit Feuchtigkeit beladene Prozessluft abgekühlt und anschließend zu einem Abluftausgang 15 geführt wird. Die abgeschiedene Feuchtigkeit wird in einem Kondensatauffangbehälter 9 aufgefangen, von wo aus sie beispielsweise durch Abpumpen mittels einer hier nicht gezeigten Kondensatpumpe entfernt werden kann.

Zur Abkühlung der mit von der Wäsche abgeführter Feuchtigkeit beladenen Prozessluft wird im Luft-Luft-Wärmetauscher 16 über den Zuluftkanal 10 dem Trockner 1 zugeführte Raumluft verwendet. Diese Zuluft wird durch die warme, mit Feuchtigkeit beladene Prozessluft und vor dem Eintritt in die Trommel 2 noch mittels der Heizung 11 erwärmt.

Die Trommel 2 wird bei der in Fig. 1 gezeigten Ausführungsform am hinteren Boden mittels eines Drehlagers und vorne mittels eines Lagerschildes 17 gelagert, wobei die Trommel 2 mit einer Krempe auf einem Gleitstreifen 18 am Lagerschild 17 aufliegt und so am vorderen Ende gehalten wird. Ein Motor 3 treibt die Trommel 2 an. Die Steuerung des Ablufttrockners 1 erfolgt über eine Programmsteuerung 4, die vom Benutzer über eine Bedieneinheit 20 geregelt werden kann. 36 bedeutet ein Anzeigemittel zur Wiedergabe der Information über ein eventuell stattfindendes erfindungsgemäßes Reinigungsverfahren. Bei Feststellung eines schlechten Geruchs durch einen Geruchssensor (hier nicht dargestellt, aber vgl. Bezugszeichen 5 in Figur 2) kann beispielsweise eine Leuchtdiode 39, die elektromagnetische Strahlung im Wellenlängenbereich von 380 bis 400 nm emittiert, angeschaltet werden. Durch diese elektromagnetische Strahlung (z.B. UV-B-Strahlung) werden an dem als Photokatalysator aktivierten Titandioxid enthaltenden Schicht Sauerstoffradikale erzeugt, welche Verunreinigungen zersetzen können.

Bei der in Fig. 1 gezeigten Ausführungsform befinden sich Leuchtdioden 39, welche die zur Aktivierung des Photokatalysators nötige Photoquelle 39 bilden, an der Tür 19 sowie an der Trommelrückwand 37.

Figur 2 ist insbesondere eine schematische Darstellung der vorliegend relevanten Teile einer Waschmaschine 6, in der ein entsprechendes Verfahren durchgeführt werden kann. Die Waschmaschine 6 weist einen Laugenbehälter 7 auf, in dem eine Trommel 2 drehbar gelagert ist und durch einen Motor 3 betrieben werden kann. Die Drehachse 31 der Trommel 2 ist um einen kleinen Winkel (z.B. 13°) aus der Horizontalen nach vorne oben gerichtet, so dass man einen leichteren Zugang und Einblick in das Innere der Trommel 2 hat. Durch diese Anordnung wird im Zusammenwirken mit besonders geformten Wäschemitnehmern 24 und Schöpfeinrichtungen 25 für die Waschlauge 23 an der Innenfläche des Trommelmantels außerdem auch eine Intensivierung der Durchflutung der Wäsche 32 mit Waschlauge erreicht.

Die urgeformten Wäschemitnehmer 24 sowie eine Innenwand 40 des ebenfalls urgeformten Laugenbehälters 7 weisen Oberflächen 38 auf, die wiederum Titandioxid in der Anatas-Modifikation enthalten. Zur Erzeugung von Sauerstoffradikalen an diesen Schichten 38 sind ebenfalls an der Innenwand 40 des Laugenbehälters 7 wie auch in der Trommel 2 Leuchtdioden 39 angeordnet, die elektromagnetische Strahlung im Wellenlängenbereich von 380 bis 400 nm emittieren.

Die Waschmaschine 6 weist zudem ein Laugenzulaufsystem auf, das eine Wasseranschlussarmatur für das Hauswassernetz 28, ein elektrisch steuerbares Ventil 29 und eine Zuleitung 27 zum Laugenbehälter 7 umfasst, die über eine Einspülschale 30 geführt ist, aus der das Zulaufwasser Waschmittelportionen in den Laugenbehälter 7 transportieren kann. Eine Dosiervorrichtung 26 ermöglicht im Zusammenwirken mit dem Hauswassernetz 28 die Zuführung von Weichspüler in den Laugenbehälter 7. Außerdem befindet sich im Laugenbehälter 7 eine Heizeinrichtung 34. Das Ventil 29 wie auch die Heizeinrichtung 34 können durch eine Steuereinrichtung ("Programmsteuerung") 4 in Abhängigkeit von einem Programmablaufplan gesteuert werden, der an ein Zeitprogramm und/oder an das Erreichen von gewissen Messwerten von Parametern wie Laugenniveau, Laugentemperatur, Drehzahl der Trommel 2 usw. innerhalb der Waschmaschine 6 gebunden sein kann. 33 bedeutet einen Sensor für die Messung des hydrostatischen Druckes p im Laugenbehälter 7. 8 bedeutet eine Pumpe für die im Laugenbehälter 7 befindliche Flüssigkeit.

Bei der Ausführungsform von Fig. 2 ist ein Geruchssensor 5 im Laugenbehälter 7 angeordnet. 36 bedeutet ein Anzeigemittel zur Wiedergabe der Information über ein eventuell stattfindendes oder durchzuführendes Reinigungsverfahren.

Schließlich weist das Hausgerät in der Ausführungsform von Fig. 2 einen optischen Sensor 41,42 mit einer Lichtquelle 41 und einem Lichtempfänger 42 auf, wobei die Lichtquelle 41 eine Leuchtdiode 41 umfasst, die elektromagnetische Strahlung im Wellenlängenbereich von 380 bis 400 nm emittiert. Die Umgebung des Lichtempfängers 42 weist wiederum Titandioxid als Photokatalysator in Oberflächen 38 urgeformter Bauteile wie dem Laugenbehälter 7 auf. Die Leuchtdiode 41 emittiert elektromagnetische Strahlung im Wellenlängenbereich von 380 bis 400 nm derart, dass sie die Titandioxid enthaltende Oberfläche 38 zwecks Aktivierung des Photokatalysators bestrahlen kann.

Figur 3 zeigt einen Schnitt durch ein urgeformtes, namentlich durch Spritzgießen erzeugtes Bauteil 7 in Form des Laugenbehälters 7 der Figur 2, um die Oberfläche 38, die den Photokatalysator 43 enthält, deutlich darzustellen. Dabei ist der Photokatalysator 43 ein Füllstoff 43 in dem ersten Werkstoff 44, wobei der erste Werkstoff 44 ein mit dem Füllstoff 43 gefüllter erster polymerer Werkstoff 44 ist. Das Polymer, insbesondere ein durch Spritzgießen oder alternativ Formpressen verarbeiteter Thermoplast 44 wie Polypropylen, bildet dabei eine kontinuierlich Matrix, in welcher der Füllstoff 43 dispergiert ist.

Der erste Werkstoff 4 bildet eine Schicht an der Oberfläche 38 und überdeckt einen zweiten Werkstoff 45. Dieser ist vorliegend ein mit einem anderen Füllstoff 46 gefülltes thermoplastisches Polymer, welches dem Polymer des ersten Werkstoffes gleichen kann oder zumindest so ähnlich ist, dass es in demselben Spritzgießwerkzeug wie der erste Werkstoff 44 verarbeitbar ist. Auf diese Weise kann das Bauteil 7 in einem zweistufigen Spritzgießprozess erzeugt werden, bei dem zunächst der erste Werkstoff 44 in eine vorgegebene Form eingespritzt wird und diese nur teilweise ausfüllt, worauf nachfolgend und vor einer völligen Verfestigung des ersten Werkstoffs 44 der zweite Werkstoff 45 bis zum vollständigen Ausfüllen der Form eingespritzt wird und unter dem ersten Werkstoff 44, welcher eine Schicht an der Oberfläche 38 bildet, zu liegen kommt. Auf die Form der Trennfläche 47 zwischen dem ersten Werkstoff 44 und dem zweiten Werkstoff 45 kommt es dabei weniger an, zumal der Photokatalysator 38 nur dann katalytisch aktiv werden kann, wenn er direkt an der Oberfläche 38 liegt und somit gleichermaßen Sauerstoff und aktivierender elektromagnetischer Strahlung aussetzbar ist. Aus diesem Grunde muss auch der Füllstoff 46 im zweiten Werkstoff 45 nicht photokatalytisch wirksam sein; der Füllstoff 46 kann entsprechend üblicher Praxis Talkum und dergleichen sein, es ist auch denkbar, Glasfasern zu verwenden, um dem zweiten Werkstoff 45 eine höhere Festigkeit oder andere günstigere Eigenschaften als dem ersten Werkstoff 44 zu geben. Es mag auch lediglich gewünscht sein, ein billigeres Material als Titandioxid im zweiten Werkstoff 45 zu verwenden.

In jedem Falle kommt als Photokatalysator 43 ein halbleitendes Mineral 43 zum Einsatz, und zwar vorliegend ein im Wesentlichen aus Titandioxid 43 bestehendes Mineral 43, in dem das Titandioxid 43 in der Modifikation von Anatas vorliegt und zusätzlich eine Dotierung aus Kohlenstoff aufweist. Deshalb kann zur Aktivierung des Photokatalysators sichtbares Licht verwendet werden, was die Nutzung billigerer Photoquellen 39, 41 als Leuchtdioden, die UV-Licht erzeugen, ermöglicht. Das Mineral 43 liegt in Form von Partikeln 43 mit einem mittleren Durchmesser d kleiner oder gleich 10 nm im ersten Werkstoff 44 vor.

## Patentansprüche

1. Hausgerät (1,6) mit mindestens einem Bauteil (7, 21, 24), welches eine durch eine organische Verschmutzung belastbare Oberfläche (38) aufweist, welche Oberfläche (38) einen Photokatalysator (43) aufweist und welcher Oberfläche (38) eine Photoquelle (39, 41) zum Bestrahlen des Photokatalysators (43) mit einer aktivierenden elektromagnetischen Strahlung zugeordnet ist, **dadurch gekennzeichnet, dass** die Oberfläche (38) von einem urgeformten ersten Werkstoff (44) gebildet ist, in welchem der Photokatalysator (43) dispergiert ist.

2. Hausgerät (1, 6) nach Anspruch 1, bei dem der Photokatalysator (43) ein Füllstoff (43) in dem ersten Werkstoff (44) ist.

3. Hausgerät nach Anspruch 2, bei dem der erste Werkstoff (44) ein mit dem Füllstoff (43) gefüllter erster polymerer Werkstoff (44) ist.

4. Hausgerät (1, 6) nach Anspruch 3, bei dem der erste Werkstoff (44) ein mit dem Füllstoff (43) gefüllter Thermoplast (44) ist.

5. Hausgerät (1, 6) nach einem der vorigen Ansprüche, bei dem der erste Werkstoff (44) eine Schicht an der Oberfläche (38) bildet und einen zweiten Werkstoff (45) überdeckt.

6. Hausgerät (1, 6) nach einem der vorigen Ansprüche, bei dem der Photokatalysator (43) ein halbleitendes Mineral (43) ist.

7. Hausgerät (1, 6) nach Anspruch 6, bei dem der Photokatalysator (43) ein im Wesentlichen aus Titandioxid (43) bestehendes Mineral (43) ist.

8. Hausgerät (1, 6) nach Anspruch 7, bei dem das Titandioxid (43) in der Modifikation von Anatas vorliegt.

9. Hausgerät (1, 6) nach einem der Ansprüche 7 und 8, bei dem das Titandioxid (43) eine Dotierung aus Kohlenstoff aufweist.

10. Hausgerät (1, 6) nach einem der Ansprüche 6 bis 9, **dadurch gekennzeichnet, dass** das Mineral (43) aus Partikeln (43) mit einem mittleren Durchmesser d kleiner oder gleich 10 nm besteht.

11. Hausgerät (1, 6) nach einem der vorigen Ansprüche, bei dem die Photoquelle (39, 41) eingerichtet ist zum Aussenden der elektromagnetischen Strahlung in einem Wellenlängenbereich umfassend sichtbares Licht und ultraviolettes Licht.

12. Hausgerät (1, 6) nach Anspruch 11, bei dem die Photoquelle (39, 41) eingerichtet ist zum Aussenden der elektromagnetischen Strahlung im Wellenlängenbereich von 380 nm bis 400 nm.

13. Hausgerät (1, 6) nach einem der vorigen Ansprüche, bei dem die Photoquelle (39, 41) eine Anordnung mit zumindest einer Leuchtdiode (39) ist.

14. Hausgerät (1, 6) nach Anspruch 13, bei dem die Photoquelle (39, 41) mindestens sechs Leuchtdioden (39) aufweist.

15. Hausgerät (1, 6) nach einem der vorigen Ansprüche, welches ein Geschirrspülgerät oder ein Wäschebehandlungsgerät (1,6) ist.

16. Hausgerät (1, 6) nach Anspruch 15, welches ein Wäschebehandlungsgerät (1, 6) aus der Gruppe Waschmaschine, Trockner und Waschtrockner ist.

17. Hausgerät (1, 6) nach einem der Ansprüche 15 und 16, welches zumindest ein mit einer Waschlauge beaufschlagbares Bauteil (7, 21, 24) aufweist, welches Bauteil (7, 21, 24) die Oberfläche (38) aufweist, in der der Photokatalysator (43) dispergiert ist.

18. Hausgerät (1, 6) nach Anspruch 17, bei dem das Bauteil (7, 21, 24) ausgewählt ist aus der Gruppe umfassend Wäschemitnehmer (21, 24) und Laugenbehälter (7).

19. Hausgerät (1, 6) nach einem der Ansprüche 15 bis 18 bei dem die Photoquelle (39, 41) im Bereich einer Zugangsöffnung (19) angeordnet ist.

20. Hausgerät (1, 6) nach einem der vorigen Ansprüche, **dadurch gekennzeichnet, dass** es einen optischen Sensor (41, 42) mit einer zugeordneten Lichtquelle (41) und einem Lichtempfänger (42) aufweist, wobei die zugeordnete Lichtquelle (41) die Photoquelle (39, 41) ist.

21. Hausgerät (1, 6) nach einem der vorigen Ansprüche, **dadurch gekennzeichnet, dass** es einen Geruchssensor (5) aufweist.

22. Verfahren zur Reinigung einer durch eine organische Verschmutzung belastbare Oberfläche (38) mindestens eines Bauteils (7, 21, 24) eines Hausgeräts (1, 6), in welcher Oberfläche (38) ein Photokatalysator (43) dispergiert und welcher Oberfläche eine Photoquelle (39, 41) zum Bestrahlen des Photokatalysators (43) mit einer aktivierenden elektromagnetischen Strahlung zugeordnet ist, und wobei die Oberfläche (38) von einem urgeformten ersten Werkstoff (44) gebildet ist, **dadurch gekennzeichnet, dass** der Photokatalysator (43) von der Photoquelle (39, 41) durch Bestrahlen mit elektromagnetischer Strahlung aktiviert wird.

## Claims

1. Domestic appliance (1, 6) with at least one component (7, 21, 24), which has a surface (38) loadable by an organic contaminant, which surface (38) comprises a photocatalyst (43) and with which surface (38) a photo source (39, 41) for irradiation of the photocatalyst (43) with an activating electromagnetic radiation is associated, **characterised in that** the surface (38) is formed by a primary-formed first material (44) in which the photocatalyst (43) is dispersed.

2. Domestic appliance (1, 6) according to claim 1, in which the photocatalyst (43) is a filler (43) in the first material (44).

3. Domestic appliance (1, 6) according to claim 2, in which the first material (44) is a first polymer material (44) filled with the filler (43).

4. Domestic appliance (1, 6) according to claim 3, in which the first material (44) is a thermoplastic (44) filled with the filler (43).

5. Domestic appliance (1, 6) according to any one of the preceding claims, in which the first material (44) forms a layer at the surface (38) and covers a second material (45).

6. Domestic appliance (1, 6) according to any one the preceding claims, in which the photocatalyst (43) is a semiconductive mineral (43).

7. Domestic appliance (1, 6) according to claim 6, in which the photocatalyst (43) is a mineral (43) substantially consisting of titanium dioxide (43).

8. Domestic appliance (1, 6) according to claim 7, in which the titanium dioxide (43) is present in the modification of anatase.

9. Domestic appliance (1, 6) according to one of claims 7 and 8, in which the titanium dioxide (43) has a doping of carbon.

10. Domestic appliance (1, 6) according to any one of claims 6 to 9, **characterised in that** the mineral (43) consists of particles (43) with a mean diameter d smaller than or equal to 10 nm.

11. Domestic appliance (1, 6) according to any one of the preceding claims, in which the photo source (39, 41) is arranged for emitting electromagnetic radiation in a wavelength range comprising visible light and ultraviolet light.

12. Domestic appliance (1, 6) according to claim 11, in which the photo source (39, 41) is arranged for emitting the electromagnetic radiation in the wavelength of 380 nm to 400 nm.

13. Domestic appliance (1, 6) according to any one of the preceding claims, in which the photo source (39, 41) is an arrangement with at least one light-emitting diode (39).

14. Domestic appliance (1, 6) according to claim 13, in which the photo source (39, 41) comprises at least six light-emitting diodes (39).

15. Domestic appliance (1, 6) according to any one of the preceding claims, which is a dishwasher or a laundry treatment appliance (1, 6).

16. Domestic appliance (1, 6) according to claim 15, which is a laundry treatment appliance (1, 6) from the group of washing machine, dryer and washer-dryer.

17. Domestic appliance (1, 6) according to one of claims 15 and 16, which comprises at least one component (7, 21, 24) able to be acted on by a washing solution, which component (7, 21, 24) has the surface (38) in which the photocatalyst (43) is dispersed.

18. Domestic appliance (1, 6) according to claim 17, in which the component (7, 21, 24) is selected from the group comprising laundry entrainers (21, 24) and solution container (7).

19. Domestic appliance (1, 6) according to any one of claims 15 to 18, in which the photo source (39, 41) is arranged in the region of an access opening (19).

20. Domestic appliance (1, 6) according to any of the preceding claims, **characterised in that** it comprises an optical sensor (41, 42) with an associated light source (41) and a light receiver (42), wherein the associated light source (41) is the photo source (39, 41).

21. Domestic appliance (1, 6) according to any one of the preceding claims, **characterised in that** it comprises an odour sensor (5).

22. Method for cleaning a surface (38), which is loadable by an organic contaminant, of at least one component (7, 21, 24) of a domestic appliance (1, 6), in which surface (38) of a photocatalyst (43) is dispersed and with which surface a photo source (39, 41) for irradiation of the photocatalyst (43) with an activating electromagnetic radiation is associated, and wherein the surface (38) is formed by a primary-formed first material (44), **characterised in that** the photocatalyst (43) is activated by the photo source (39, 41) through irradiation with electromagnetic radiation.

## Revendications

1. Appareil ménager (1, 6) constitué d'au moins un composant (7, 21, 24) pourvu d'une surface (38) susceptible d'être exposée à des salissures organiques, ladite surface (38) comprenant un photocatalyseur (43) et une source de lumière (39, 41), destinée à exposer le photocatalyseur (43) à un rayonnement électromagnétique activateur, étant associée à ladite surface (38), **caractérisé en ce que** la surface (38) est formée par un premier matériau (44) soumis à un façonnage initial et dans lequel le photocatalyseur (43) est dispersé.

2. Appareil ménager (1, 6) selon la revendication 1, dans lequel le photocatalyseur (43) est un matériau de charge (43) dans le premier matériau (44).

3. Appareil ménager selon la revendication 2, dans lequel le premier matériau (44) est un premier matériau polymère (44) rempli du matériau de charge (43).

4. Appareil ménager (1, 6) selon la revendication 3, dans lequel le premier matériau (44) est un thermoplastique (44) rempli du matériau de charge (43).

5. Appareil ménager (1, 6) selon l'une des revendications précédentes, dans lequel le premier matériau (44) constitue une couche sur la surface (38) et couvre un deuxième matériau (45).

6. Appareil ménager (1, 6) selon l'une des revendications précédentes, dans lequel le photocatalyseur (43) est un minéral semi-conducteur (43).

7. Appareil ménager (1, 6) selon la revendication 6, dans lequel le photocatalyseur (43) est un minéral (43) essentiellement composé de dioxyde de titane (43).

8. Appareil ménager (1, 6) selon la revendication 7, dans lequel le dioxyde de titane (43) se trouve dans la modification anatase.

9. Appareil ménager (1, 6) selon l'une des revendications 7 et 8, dans lequel le dioxyde de titane (43) présente une dotation carbone.

10. Appareil ménager (1, 6) selon l'une des revendications 6 à 9, **caractérisé en ce que** le minéral (43) se compose de particules (43) dont le diamètre moyen d est inférieur ou égal à 10 nm.

11. Appareil ménager (1, 6) selon l'une des revendications précédentes, dans lequel la source de lumière (39, 41) est prévue afin d'émettre le rayonnement électromagnétique dans une plage de longueurs d'ondes comprenant la lumière visible et la lumière ultraviolette.

12. Appareil ménager (1, 6) selon la revendication 11, dans lequel la source de lumière (39, 41) est prévue afin d'émettre le rayonnement électromagnétique dans une plage de longueurs d'ondes de 380 nm à 400 nm.

13. Appareil ménager (1, 6) selon l'une des revendications précédentes, dans lequel la source de lumière (39, 41) est un agencement avec au moins une diode luminescente (39).

14. Appareil ménager (1, 6) selon la revendication 13, dans lequel la source de lumière (39, 41) présente au moins six diodes luminescentes (39).

15. Appareil ménager (1, 6) selon l'une des revendications précédentes, lequel est un lave-vaisselle ou un appareil de traitement du linge (1, 6).

16. Appareil ménager (1, 6) selon la revendication 15, lequel est un appareil de traitement du linge (1, 6) issu du groupe composé par les lessiveuses, les séchoirs et les séchoirs à linge.

17. Appareil ménager (1, 6) selon l'une des revendications 15 et 16, lequel présente au moins un composant (7, 21, 24) susceptible d'être alimenté en lessive, lequel composant (7, 21, 24) présente la surface (38) dans laquelle le photocatalyseur (43) est dispersé.

18. Appareil ménager (1, 6) selon la revendication 17, dans lequel le composant (7, 21, 24) est sélectionné parmi le groupe comprenant les éléments d'entraînement du linge (21, 24) et les cuves à lessive (7).

19. Appareil ménager (1, 6) selon l'une des revendications 15 à 18 dans lequel la source de lumière (39, 41) est disposée dans la zone d'un orifice d'accès (19).

20. Appareil ménager (1, 6) selon l'une des revendications précédentes, **caractérisé en ce qu'**il présente un capteur optique (41, 42) auquel une source lumineuse (41) est affectée et un récepteur de lumière (42), dans lequel la source lumineuse affectée (41) est la source de lumière (39, 41).

21. Appareil ménager (1, 6) selon l'une des revendications précédentes, **caractérisé en ce qu'**il présente un capteur d'odeur (5).

22. Procédé de nettoyage d'une surface (38) susceptible d'être exposée à des salissures organiques d'au moins un composant (7, 21, 24) d'un appareil ménager (1, 6), dans laquelle surface (38) un photocatalyseur (43) est dispersé et à laquelle surface une source de lumière (39, 41) afin d'exposer le photocatalyseur (43) à un rayonnement électromagnétique activateur est associée, et dans lequel la surface (38) est formée par un premier matériau (44) soumis à un façonnage initial, **caractérisé en ce que** le photocatalyseur (43) est activé par la source de lumière (39, 41) via l'exposition par rayonnement électromagnétique.
